# EUROPÄISCHE PATENTSCHRIFT

(11) **EP 2 522 391 B1**
(45) Veröffentlichungstag und Bekanntmachung des Hinweises auf die Patenterteilung: **03.08.2016**
(21) Anmeldenummer: 12164376.1
(22) Anmeldetag: 17.04.2012
(51) Int. Cl.: A61N 1/37, A61N 1/372, A61N 1/08

(54) **Implantierbares medizinisches Gerät mit Elektrodenfehlererkennung**
Implantable medical device with electrode fault detection
Appareil médical implantable avec détection des pannes d'électrode

(30) Priorität: 10.05.2011 US 201161484245 P
(43) Veröffentlichungstag der Anmeldung: 14.11.2012
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- WO-A1-2012/057863
- US-A- 5 683 430
- US-A1- 2009 326 600
- US-B1- 7 389 144

## Beschreibung

Die Erfindung betrifft ein implantierbares medizinisches Gerät mit Mitteln zum Erkennen von Elektrodendefekten. Die Erfindung betrifft insbesondere ein implantierbares medizinisches Gerät mit einem Elektrodenleitungsanschluss mit wenigstens einem elektrischen Kontakt zum Anschließen einer Elektrodenleitung.

Bekannt sind Implantate, die zur Elektrodenintegritätsprüfung eine Messung von Elektrodenimpedanzen, Wahrnehmungsamplituden, Stimulationsreizschwellen oder Wahrnehmungssignalmustern durchführen. Jedoch sind die bekannten Verfahren nicht sensitiv genug, um alle Elektrodendefekte bereits im Anfangsstadium erfassen zu können, d.h. diese zu einem Zeitpunkt zu erkennen, wenn die Therapie- und Diagnosefunktionen noch nicht eingeschränkt sind.

In Farwell et al, Inappropriate implantable cardioverter defibrillator shocks in fractured Sprint Fidelis leads associated with 'appropriate' interrogation; Europace (2008) 10, 726-728*,* ist eine Fallstudie eines defekten Elektrodensystems beschrieben, bei dem die elektromagnetische Einstrahlung der Programmiertelemetrie zu einer inadäquaten Schockabgabe geführt hat. In dem zitierten Aufsatz wird sehr anschaulich die Einkopplung einer elektromagnetischen Welle durch ein Programmiergerät in ein defektes, aber bislang unauffälliges Elektrodensystem dargestellt. Hier führt die Spulentelemetrie des Programmiergerätes zu einer typischen Einkopplung in die als Antenne wirkende Elektrodenleitung. Da durch den vorliegenden Defekt der Elektrodenleitung deren Antennencharakteristik im System nachhaltig verändert ist, wird diese Einkopplung als Arrhythmie-Signal vom elektronischen Implantat wahrgenommen und löst eine Schockabgabe aus. Bei einer intakten Elektrodenleitung hat die Einstrahlung der Programmiertelemetrie hingegen keine Störsignale in der Eingangsstufe des ICD zur Folge.

US 7,389,144 beschreibt ein implantierbares medizinisches Gerät mit einem Elektrodenleitungsanschluss mit wenigstens einem elektrischen Kontakt zum Anschließen einer Elektrodenleitung und mit einer mit dem Elektrodenleitungsanschluss verbundenen Analyseeinheit die ausgebildet ist, ein an dem wenigstens einen elektrischen Kontakt als Reaktion auf eine bekannte elektromagnetische Einstrahlung anliegendes Antwortsignal zu erfassen und auszuwerten.

Vor diesem Hintergrund liegt der Erfindung die Aufgabe zugrunde, eine Elektrodenfehlererkennung frühzeitiger und spezifischer als bislang durchführen zu können.

Erfindungsgemäß wird diese Aufgabe durch ein implantierbares medizinisches Gerät mit einem Elektrodenleitungsanschluss mit wenigstens einem elektrischen Kontakt zum Anschließen einer Elektrodenleitung gelöst, das eine mit dem Elektrodenleitungsanschluss verbundene Analyseeinheit aufweist, die ausgebildet ist, ein an dem wenigstens einen elektrischen Kontakt als Reaktion auf eine bekannte elektromagnetische Einstrahlung anliegendes Antwortsignal zu erfassen und auszuwerten und die sich dadurch auszeichnet, dass die Analyseeinheit zusätzlich zum Elektrodenleitungsanschluss mit einer weiteren Empfangseinrichtung für eine eingestrahlte elektromagnetische Welle verbunden und ausgebildet ist, das über den Elektrodenleitungsanschluss erfasste Antwortsignal mit einem über die weitere Empfangseinrichtung empfangenen zweiten Antwortsignal zu vergleichen.

Die Analyseeinheit ist somit in der Lage, eine an den Anschluss angeschlossene Elektrodenleitung anhand von deren Reaktion auf eine in die Elektrodenleitung eingekoppelte elektromagnetische Interferenz bekannter Quelle zu charakterisieren, und es ergibt sich ein implantierbares elektronisches System, verbunden oder zu verbinden mit mindestens einer Elektrodenleitung und mit einer Analyseeinheit zur Charakterisierung der Elektrodenleitung als Reaktion auf eine in die Elektrodenleitung eingekoppelte elektromagnetische Interferenz bekannter Quelle. Auf diese Weise kann die Analyseeinheit gegebenenfalls auch erfassen, wenn aufgrund beispielsweise eines Fehlers kein Kontakt des Elektrodenleitungsanschlusses zu einer Elektrodenleitung besteht. Desweiteren kann sie die Reaktion auf die elektromagnetische Einstrahlung an der Elektrodenleitung mit einer zweiten Reaktion des Systems auf diese Einstrahlung vergleichen.

Das erfindungsgemäße implantierbare medizinische Gerät nutzt die Tatsache aus, dass Elektrodenleitungen als Antennen für eingekoppelte Signale im funktionstüchtigen Zustand eine definierte Antennencharakteristik aufweisen und diese sich im Fehlerfall nennenswert verändert und gestattet so eine frühere und zuverlässigere Erkennung von Elektrodendefekten bei elektronischen Implantaten, die mit Elektrodenleitungen verbunden sind, als dies beim Stand der Technik der Fall ist.

Vorzugsweise ist die Analyseeinheit ausgebildet, ein Antwortsignal auf eine Einstrahlung im MICS-Frequenzband zu erfassen und auszuwerten. In diesem Fall kann die elektromagnetische Einstrahlung von einem im MICS-Band arbeitenden Programmiergerät stammen. MICS steht für "Medical Implant Communication Service" und bezeichnet ein für die drahtlose Kommunikation mit medizinischen Implantaten vorgesehenes Frequenzband zwischen 402 und 405 MHz. Entsprechend kann die elektromagnetische Einstrahlung auch von einem Patientengerät stammen, welches als Relaisstation in der Nähe eines Patienten dazu dient, über das MICS-Band eine telemetrische Verbindung zum einem Implantat herzustellen und über das MICS-Band zwischen Implantat und Patientengerät ausgetauschte Daten über eine beispielsweise drahtlose oder drahtgebundene Datenverbindung an ein zentrales Service-Center weiterzuleiten oder umgekehrt Daten oder Steuerbefehle von einem Service-Center zum Implantat zu übertragen.

Alternativ oder zusätzlich kann die Analyseeinheit auch ausgebildet sein, ein Antwortsignal auf eine von einer Programmierspule eines Programmiergerätes abgegebenen Einstrahlung zu erfassen und auszuwerten. Die elektromagnetische Einstrahlung ist dann Folge einer Programmierspulentelemetrie, wie sie zwischen einem entsprechenden Programmiergerät mit einer Programmierspule und einer entsprechenden Spule im Implantat durchgeführt wird.

Allgemein ist es vorteilhaft, wenn die Analyseeinheit mit einer elektromagnetischen Strahlungsquelle verbunden oder zu verbinden ist, wobei die elektromagnetische Strahlungsquelle ausgebildet ist, eine elektromagnetische Welle auszusenden, die geeignet ist, in eine an den Elektrodenleitungsanschuss angeschlossene Elektrodenleitung einzukoppeln. Eine solche Verbindung zwischen Analyseeinheit und Strahlungsquelle kann dabei so gestaltet sein, dass die Analyseeinheit derart drahtlos mit einer elektromagnetischen Strahlungsquelle zu verbinden ist, dass die Analyseeinheit automatisch ein Aussenden einer elektromagnetischen Welle durch eine jeweilige Strahlungsquelle automatisch erfasst, das heißt, die Verbindung kann einseitig und passiv sein und auf das automatische Erkennen einer elektromagnetischen Einstrahlung, die für einen Elektrodenleitungstest geeignet ist, beschränkt sein. Genauso kann die Verbindung auch das Empfangen dezidierter Steuersignale durch die Analyseeinheit beinhalten. Die Analyseeinheit ist in diesen Fällen ausgebildet, in Reaktion auf ein Erfassen des Aussendens einer elektromagnetischen Welle durch eine jeweilige Strahlungsquelle ein Erfassen und Auswerten eines entsprechenden Antwortsignals an dem wenigstens einen Kontakt des Elektrodenleitungsanschlusses zu starten.

Besonders vorteilhaft ist es, wenn die Analyseeinheit ausgebildet ist, eine von mehreren potenziellen Strahlungsquellen anhand eines von einer jeweiligen Strahlungsquelle ausgesandten Signals oder anhand einer jeweiligen, von einer jeweiligen Strahlungsquelle ausgesandten elektromagnetischen Welle zu erkennen und ein jeweiliges Antwortsignal für eine jeweils erkannte Strahlungsquelle spezifisch auszuwerten.

Die Strahlungsquelle kann auch in das implantierbare medizinische Gerät integriert sein, d.h. die Strahlungsquelle zum Erzeugen einer elektromagnetischen Welle, die mit der Elektrodenleitung interferiert, ist Bestandteil des implantierbaren medizinischen Gerätes selbst und die elektromagnetische Einstrahlung wird im Falle eines Elektrodenleitungstests durch das Implantat selbst erzeugt.

Vorzugsweise weist das implantierbare medizinische Gerät eine Steuereinheit zum Steuern des implantierbaren medizinischen Gerätes in verschiedenen Betriebsmodi auf, wobei die Steuereinheit mit der Analyseeinheit verbunden und ausgebildet ist, das implantierbare medizinische Gerät für die Dauer des Erfassens eines Antwortsignals durch die Analyseeinheit in einen entsprechenden spezifischen Betriebsmodus zu schalten. Für den Zeitraum der Einstrahlung der elektromagnetischen Welle kann dann das Implantat in einen dafür sicher geltenden Modus geschaltet werden.

Die zweite Reaktion des Systems wird vorzugsweise von einer von der Elektrodenleitung unabhängigen "Antenne" als weiterer Empfangseinrichtung empfangen. Hierbei kann die weitere Empfangseinrichtung eine Programmierempfangsspule des implantierbaren medizinischen Gerätes zum Empfangen von Programmierbefehlen eines externen Programmiergerätes sein oder eine Antenne für die MICS-Band-Telemetrie.

In jedem Fall ist eine Analyseeinheit bevorzugt, die ausgebildet ist, ein jeweiliges Antwortsignal in Bezug auf ein gespeichertes oder parallel erfasstes Referenzsignal auszuwerten und ein einen Elektrodenleitungsfehler anzeigendes Signal zu erzeugen, wenn das jeweilige Antwortsignal in wenigstens einer Hinsicht um mehr als ein vorgegebenes Maß abweicht. Das Referenzsignal kann in einem Speicher des implantierbaren medizinischen Gerätes gespeichert sein oder ein über die weitere Empfangseinrichtung empfangenes Referenzsignal sein.

Die Erfindung soll nun anhand von Beispielen mit Bezugnahme auf die Figuren näher erläutert werden. Von den Figuren zeigt:
- Fig. 1:: Die Auswirkung einer fehlerhaften Elektrodenleitung anhand eines beispielhaften Elektrokardiogramms;
- Fig. 2:: ein Gesamtsystem aus Implantat und externem Gerät als Strahlungsquelle;
- Fig. 3:: ein vereinfachtes Blockschaltbild eines erfindungsgemäßen implantierbaren medizinischen Gerätes;
- Fig. 4:: ein Ablaufdiagramm zur Erläuterung der Funktionsweise des erfindungsgemäßen implantierbaren medizinischen Gerätes aus Fig. 3; und
- Fig. 5:: ein Testsignal.

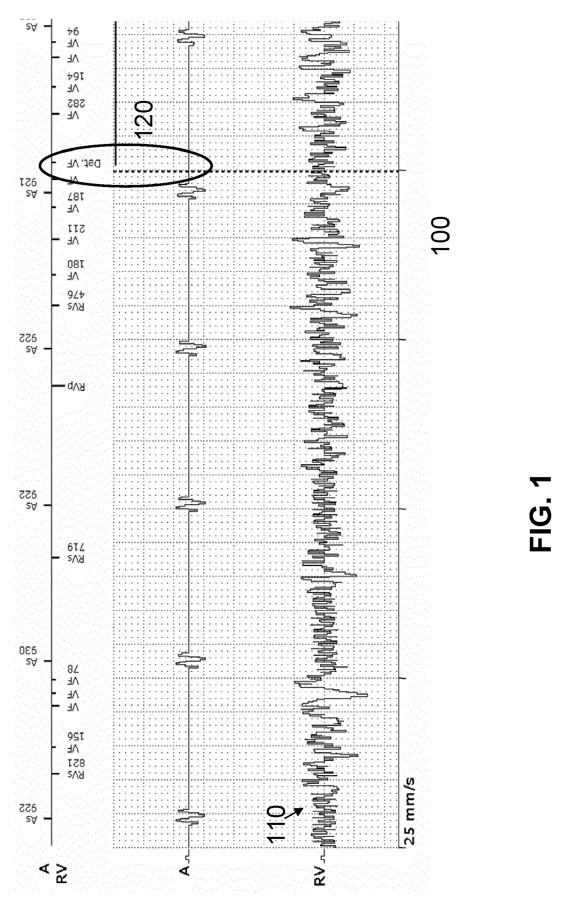

In der Fig. 1 ist ein Beispiel eines Elektrokardiogramms 100 dargestellt, das eine durch eine Programmierspulentelemetrie (also ein Programmiergerät oder ein externes Patientengerät) in eine defekte Elektrodenleitung eines implantierbaren Defibrillators (ICD) eingekoppelte Störung darstellt. Diese eingekoppelte Störung äußert sich in dem von einer rechtsventrikulären Wahrnehmungselektrode (RV) aufgenommenen rechtsventrikulären intrakardialen Elektrokardiogramm (IEGM) 110 und damit im rechtsventrikulären IEGM-Kanal 110. Die Störung führt zu einer falschen Erkennung eines ventrikulären Flimmerns (siehe Bezugszeichen 120) und damit zum Start des Ladevorganges eines Kondensators zur Bereitstellung der Energie für einen Defibrillationsschock.

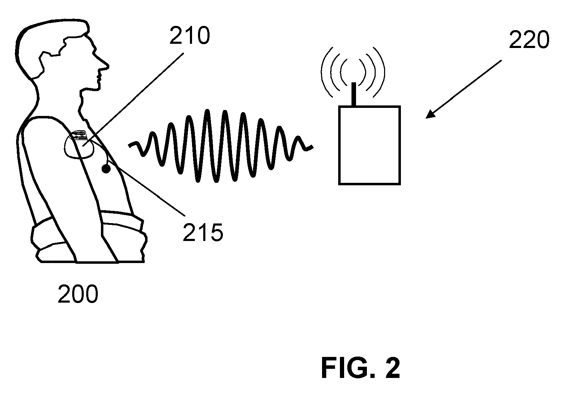

In der Fig. 2 ist ein Gesamtsystem dargestellt mit einem implantierbaren medizinischen Gerät 210 - im Folgenden auch als Implantat bezeichnet - und einem externen Programmiergerät oder Patientengerät 220. Die Figur zeigt einen Patienten 200 mit dem elektronischen Implantat 210 und daran angeschlossener Elektrodenleitung 215. In die Elektrodenleitung 215 wird beispielsweise im Falle einer telemetrischen Datenübertragung vom Patienten- oder Programmiergerät 220 eine definierte elektromagnetische Einstrahlung durch das Patienten- oder Programmiergerät 220 als dem System bekannte Strahlungsquelle eingekoppelt. Eine solche Strahlungsquelle kann z.B. das Programmiergerät oder das Patientengerät zur Home Monitoring-Überwachung sein. Idealerweise wird als elektromagnetische Einstrahlung eine elektromagnetische Welle mit einer zur Kommunikation ohnehin notwendigen Trägerfrequenz genutzt, ebenso können aber auch beliebige andere Frequenzen zur Elektrodendiagnostik abgestrahlt werden.

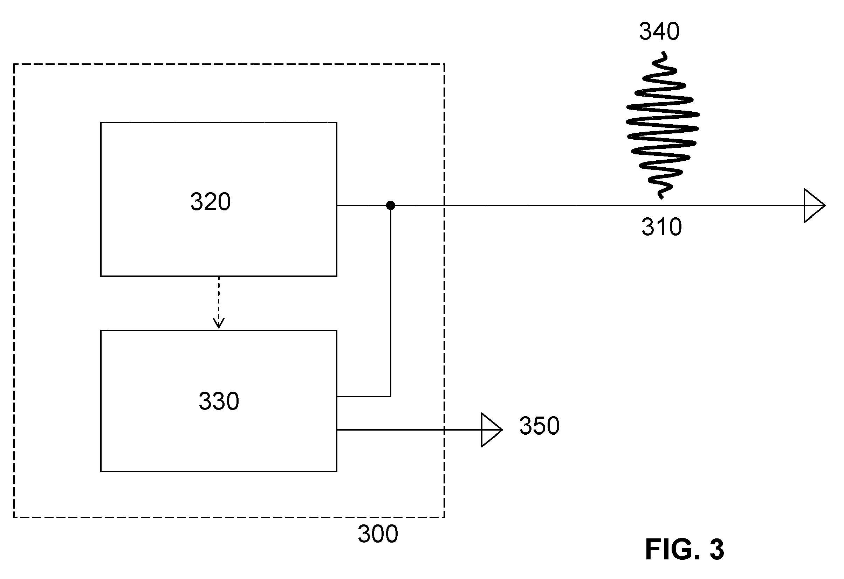

In der Fig. 3 ist ein vereinfachtes Blockschaltbild des erfindungsgemäßen Implantates 300 dargestellt, bei dem die üblichen Bestandteile bekannter implantierbarer Herzstimulatoren, wie Wahrnehmungs-, Stimulations-, Steuer- und Auswertungseinheiten, der Einfachheit halber nicht dargestellt sind. Das dargestellte Implantat 300 ist mit mindestens einer Elektrodenleitung 310 verbunden. Die von der Elektrodenleitung 310 aufgenommenen Signale werden einerseits der für Therapie und Diagnostik zugeordneten Steuereinheit 320 zugeführt. Erfindungsgemäß ist zusätzlich eine zweite, mit dem Elektrodenanschluss verbundene Analyseeinheit 330 zur Auswertung der elektromagnetischen Signalantwort an einem Elektrodenleitungsanschluss vorgesehen. Diese Analyseeinheit 330 ist derart ausgebildet, dass die im fehlerfreien Zustand der Elektrodenleitung zu erwartenden Signalmodulationen am Elektrodenleitungsanschluss bei Einkopplung einer bekannten elektromagnetischen Einstrahlung 340 bekannt sind und dass folglich Abweichungen dieser Signalmodulationen als möglicher Elektrodenfehler erkannt werden können.

Das Vorhandensein einer bekannten elektromagnetischen Einstrahlung kann der Analyseeinheit 330 z.B. über den vorhandenen Kommunikationsaufbau mit dem Programmiergerät mitgeteilt werden. Beispielsweise kann das Programmiergerät ein entsprechendes Signal senden, das von der Steuereinheit 320 oder der Analyseeinheit 330 empfangen und verarbeitet wird und den Aufbau einer telemetrischen Verbindung anzeigt oder die Steuereinheit 320 signalisiert der Analyseeinheit 330 den Aufbau einer telemetrischen Verbindung mit dem Programmiergerät, sobald eine solche erfolgt. Dies ist eine bevorzugte Lösung, die in Fig. 3 durch einen gestrichelten Pfeil angedeutet ist.

Zusätzlich kann zum Empfang einer eingestrahlten elektromagnetischen Welle und zur verbesserten Auswertung des Antwortsignals eine zweite Antenne 350 des Implantates genutzt werden, die unabhängig von der zu beurteilenden Elektrodenleitung ist. Mittels dieser zweiten Antenne 350 ist es möglich, dass die Analyseeinheit 330 das über die Elektrodenleitung 310 empfangene Antwortsignal und das über die zweite Antenne 350 als Referenzantenne empfangene Referenzsignal vergleichen kann. Die Analyseeinheit 330 ist vorzugsweise so ausgebildet, dass sie die Elektrodenleitung 310 als defekt klassifiziert, falls das Verhältnis von Antwortsignal zu Referenzsignal einen bestimmten Wert übersteigt. Außerdem ist die Analyseeinheit 330 vorzugsweise so ausgebildet, dass sie ein Signal auslöst, falls das Referenzsignal zu klein - also kleiner als ein vorgegebener unterer Grenzwert - ist. Dieses Signal kann die Abgabe eines Hinweises an einen Anwender auslösen, mit dem der Anwender darauf hingewiesen wird, den Interferenzstrahler näher an das Implantat zu platzieren.

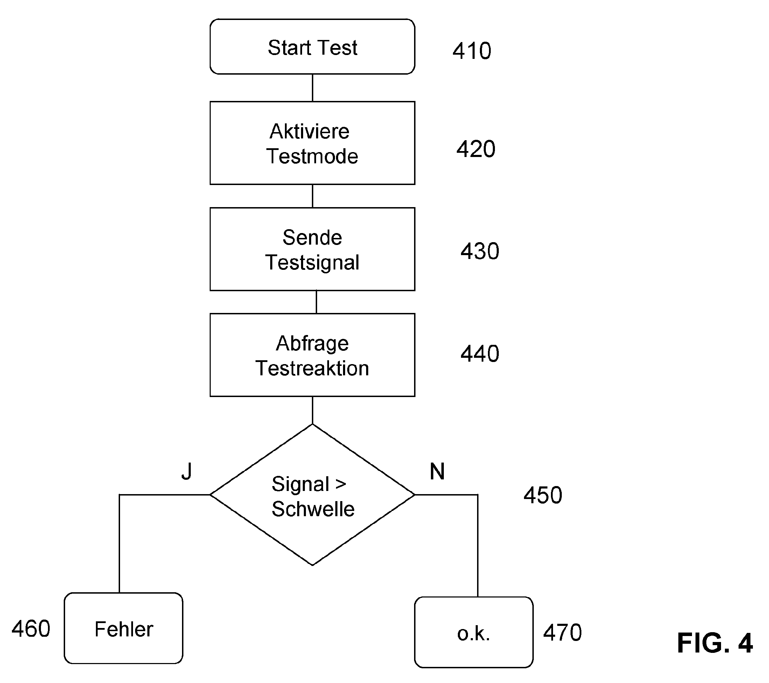

In der Fig. 4 ist ein möglicher Ablaufplan für einen Elektrodentest dargestellt. Dieser Elektrodentest wird z.B. im Rahmen einer Nachsorgeuntersuchung durch das Programmiergerät automatisch gestartet (410). Zunächst sendet dabei das Programmiergerät ein Kommando zur Testinitialisierung (420) an das Implantat, so dass z.B. die zu testende Elektrodenleitung(en) für die Therapiesteuerung deaktiviert wird, um eine unerwünschte Störung des Therapieablaufes zu vermeiden. So kann im Falle eines Zwei- oder Dreikammerschrittmachers mit einer atrialen und einer ventrikulären Elektrodenleitung beispielsweise beim Test der atrialen Elektrodenleitung der Betriebsmodus auf VVI geändert werden. In dem VVI-Betriebsmodus werden nur Signale der ventrikulären Elektrodenleitung verarbeitet.

Anschließend sendet (430) das Programmiergerät über eine geeignete Antenne die elektromagnetische Welle als Interferenzsignal an das Implantat, welches wiederum die empfangenen Signale auswertet. Am Test-Ende wird das Testergebnis aus der Analyseeinheit des Implantates ausgelesen (440) und dessen Wert beurteilt (450). Überschreitet eine Abweichung des an der Elektrodenleitung gemessenen Antwortsignals von einem gespeicherten oder empfangenen Referenzsignal als Interferenzreaktion einen Grenzwert, so wird die entsprechende Elektrodenleitung als fehlerhaft (460), andernfalls als in Ordnung (470) klassifiziert.

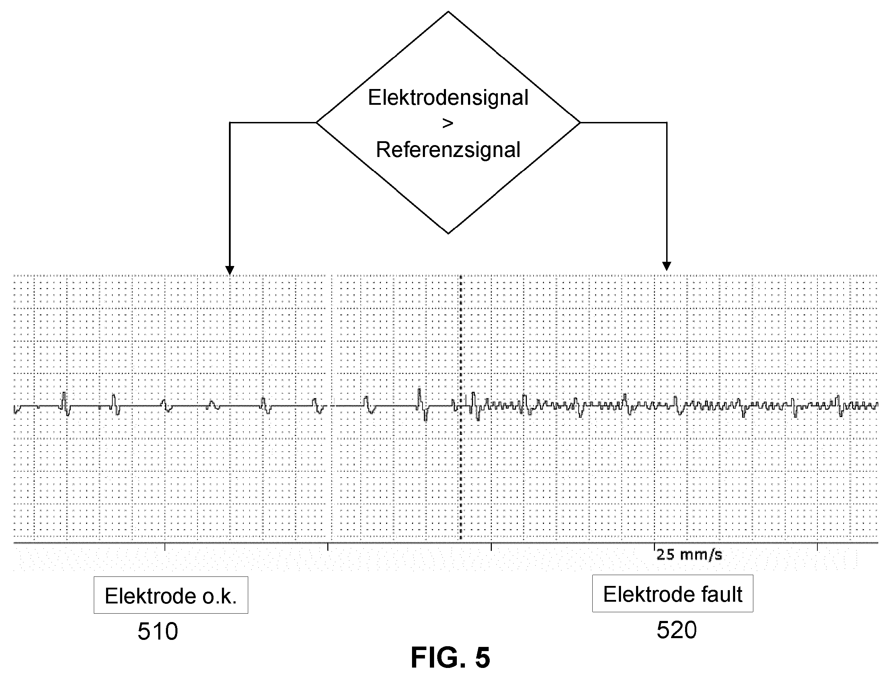

In der Fig. 5 sind beispielhaft die durch die Analyseeinheit im Implantat zu analysierenden Elektrodensignale bei Einstrahlung des Interferenzsignals dargestellt. Der linke Bildabschnitt zeigt ein Antwortsignal 510 bei einer intakten Elektrodenleitung, das eingestrahlte Signal wird nicht auf der Elektrodenleitung sichtbar. Im rechten Bildabschnitt 520 ist ein Antwortsignal dargestellt, das auftritt, wenn die Elektrodenleitung nicht intakt ist, so dass das eingestrahlte Signal der Strahlungsquelle deutlich erkennbar ist.

Die Analyseeinheit im Implantat oder dem Programmiergerät kann zur Analyse dieser Signale z.B. eine einfache Spektralanalyse einsetzen, um so das Vorhandensein der eingestrahlten Interferenz nachzuweisen.

Zusätzlich kann ein Vergleich des am Kontakt für die Elektrodenleitung nachweisbaren Antwortsignals mit einem an einer unabhängigen Antenne nachweisbaren Referenzsignal ausgewertet werden und deren Verhältnis zur Fehleranalyse verglichen werden.

Die eingestrahlten Interferenzsignale können zusätzlich eine Taktung/Impulsfolgen zur einfacheren Identifikation beinhalten.

Die erfindungsgemäße Lösung verspricht eine deutlich gesteigerte Sensitivität einer Elektrodenfehlererkennung und die Möglichkeit einer deutlich früheren Erkennung von Elektrodenfehlern. Die Methode ist für alle Arten von Elektrodenleitungen anwendbar, auch für solche, die einer "klassischen" Elektrodenintegritätsprüfung nicht zugänglich sind (z.B. Zuleitungen für einen implantierten Sensor). Die Auswertung der Signale ist dabei mit sehr einfachen Mitteln der Signalverarbeitung robust zu realisieren, so dass geringe zusätzliche Kosten bei guter Sensitivität und Spezifität zu erwarten sind.

## Patentansprüche

1. Implantierbares medizinisches Gerät (300) mit einem Elektrodenleitungsanschluss mit wenigstens einem elektrischen Kontakt zum Anschließen einer Elektrodenleitung (310) und mit einer mit dem Elektrodenleitungsanschluss verbundenen Analyseeinheit (330), die ausgebildet ist, ein an dem wenigstens einen elektrischen Kontakt als Reaktion auf eine bekannte elektromagnetische Einstrahlung (340) anliegendes Antwortsignal zu erfassen und auszuwerten
**dadurch gekennzeichnet, dass**
die Analyseeinheit (330) zusätzlich zum Elektrodenleitungsanschluss mit einer weiteren Empfangseinrichtung (350) für eine eingestrahlte elektromagnetische Welle verbunden und ausgebildet ist, das über den Elektrodenleitungsanschluss erfasste Antwortsignal mit einem über die weitere Empfangseinrichtung empfangenen zweiten Antwortsignal zur Fehleranalyse zu vergleichen.

2. Implantierbares medizinisches Gerät (300) nach Anspruch 1, **dadurch gekennzeichnet, dass** die Analyseeinheit (330) ausgebildet ist, ein Antwortsignal auf eine Einstrahlung im MICS-Frequenzband zu erfassen und auszuwerten.

3. Implantierbares medizinisches Gerät (300) nach Anspruch 1 oder 2, **dadurch gekennzeichnet, dass** die Analyseeinheit (330) ausgebildet ist, ein Antwortsignal auf eine von einer Programmierspule eines Programmiergerätes abgegebenen Einstrahlung zu erfassen und auszuwerten.

4. Implantierbares medizinisches Gerät (300) nach einem der Ansprüche 1 bis 3, **dadurch gekennzeichnet, dass** die Analyseeinheit (330) mit einer elektromagnetischen Strahlungsquelle verbunden oder zu verbinden ist, wobei die elektromagnetische Strahlungsquelle ausgebildet ist, eine elektromagnetische Welle auszusenden, die geeignet ist, in eine an den Elektrodenleitungsanschluss angeschlossene Elektrodenleitung (310) einzukoppeln.

5. Implantierbares medizinisches Gerät (300) nach Anspruch 4, **dadurch gekennzeichnet, dass** die Analyseeinheit (330) derart drahtlos mit einer elektromagnetischen Strahlungsquelle zu verbinden ist, dass die Analyseeinheit (330) automatisch ein Aussenden einer elektromagnetischen Welle durch eine jeweilige Strahlungsquelle automatisch erfasst, wobei die Analyseeinheit (330) ausgebildet ist, in Reaktion auf ein Erfassen des Aussendens einer elektromagnetischen Welle durch eine jeweilige Strahlungsquelle ein Erfassen und Auswerten eines entsprechenden Antwortsignals an dem wenigstens einen Kontakt des Elektrodenleitungsanschlusses zu starten.

6. Implantierbares medizinisches Gerät (300) nach Anspruch 5, **dadurch gekennzeichnet, dass** die Analyseeinheit (330) ausgebildet ist, eine von mehreren potenziellen Strahlungsquellen anhand eines von einer jeweiligen Strahlungsquelle ausgesandten Signals oder anhand einer jeweiligen von einer jeweiligen Strahlungsquelle ausgesandten elektromagnetischen Welle zu erkennen und ein jeweiliges Antwortsignal für eine jeweils erkannte Strahlungsquelle spezifisch auszuwerten.

7. Implantierbares medizinisches Gerät (300) nach einem der Ansprüche 1 bis 6, **gekennzeichnet durch** eine in das implantierbare medizinische Gerät integrierte Strahlungsquelle.

8. Implantierbares medizinisches Gerät (300) nach einem der Ansprüche 1 bis 7, **gekennzeichnet durch** eine Steuereinheit (320) zum Steuern des implantierbaren medizinischen Gerätes in verschiedenen Betriebsmodi, wobei die Steuereinheit (320) mit der Analyseeinheit (330) verbunden und ausgebildet ist, das implantierbare medizinische Gerät (300) für die Dauer des Erfassens eines Antwortsignals **durch** die Analyseeinheit in einen Betriebsmodus zu schalten, in dem eine jeweils zu testende Elektrodenleitung nicht zur Aufnahme therapiebeeinflussender Signale geschaltet ist.

9. Implantierbares medizinisches Gerät (300) nach einem der Ansprüche 1 bis 8, **dadurch gekennzeichnet, dass** die weitere Empfangseinrichtung (350) eine Programmierempfangsspule des implantierbaren medizinischen Gerätes zum Empfangen von Programmierbefehlen eines externen Programmiergerätes ist.

10. Implantierbares medizinisches Gerät (300) nach einem der Ansprüche 1 bis 8 , **dadurch gekennzeichnet, dass** die weitere Empfangseinrichtung (350) eine Antenne des implantierbaren medizinischen Gerätes (300) für eine Telemetrie im MICS-Frequenzband zum Datenaustausch mit einem externen Programmiergerät ist.

11. Implantierbares medizinisches Gerät (300) nach einem der Ansprüche 1 bis 10, **dadurch gekennzeichnet, dass** die Analyseeinheit (330) ausgebildet ist, ein jeweiliges Antwortsignal in Bezug auf ein gespeichertes oder parallel erfasstes Referenzsignal auszuwerten und ein einen Elektrodenleitungsfehler anzeigendes Signal zu erzeugen, wenn das jeweilige Antwortsignal um mehr als ein vorgegebenes Maß abweicht.

## Claims

1. An implantable medical device (300) comprising an electrode lead connector having at least one electrical contact for connection of an electrode lead (310), and comprising an analysing unit (330) connected to the electrode lead connector, the analysing unit being configured to detect and evaluate a response signal present at the at least one electrical contact in response to known electromagnetic radiation (340),
**characterised in that**
the analysing unit (330), in addition to being connected to the electrode lead connector, is also connected to a further receiving device (350) for an radiated electromagnetic wave and is configured to compare the response signal detected via the electrode lead connector with a second response signal received via the further receiving device for error analysis.

2. The implantable medical device (300) according to claim 1, **characterised in that** the analysing unit (330) is configured to detect and evaluate a response signal to radiation in the MICS frequency band.

3. The implantable medical device (300) according to claim 1 or 2, **characterised in that** the analysing unit (330) is configured to detect and evaluate a response signal to radiation output by a programmer coil of a programmer device.

4. The implantable medical device (300) according to any one of claims 1 to 3, **characterised in that** the analysing unit (330) is connected or connectable to a source of electromagnetic radiation, wherein the source of electromagnetic radiation is configured to transmit an electromagnetic wave which is suited to be coupled into an electrode lead (310) connected to the electrode lead connector.

5. The implantable medical device (300) according to claim 4, **characterised in that** the analysing unit (330) is wirelessly connectable to a source of electromagnetic radiation such that the analysing unit (330) automatically detects a transmission of an electromagnetic wave by a particular radiation source, wherein the analysing unit (330) is configured, in response to detection of the transmission of an electromagnetic wave by a particular radiation source, to start a detection and evaluation of a corresponding response signal at the at least one contact of the electrode lead connector.

6. The implantable medical device (300) according to claim 5, **characterised in that** the analysing unit (330) is configured to detect one of a plurality of potential radiation sources on the basis of a signal transmitted by a particular radiation source, or to detect a particular electromagnetic wave transmitted by a particular radiation source, and specifically evaluate a particular response signal for a particular radiation source that was detected.

7. The implantable medical device (300) according to any one of claims 1 to 6, **characterised by** a radiation source integrated into the implantable medical device.

8. The implantable medical device (300) according to any one of claims 1 to 7, **characterised by** a control unit (320) for controlling the implantable medical device in various operating modes, wherein the control unit (320) is connected to the analysing unit (330) and is configured, for the duration of the detection of a response signal by the analysing unit, to switch the implantable medical device (300) to an operating mode in which a particular electrode lead to be tested is not switched to receive signals that influence therapy.

9. The implantable medical device (300) according to any one of claims 1 to 8, **characterised in that** the further receiving device (350) is a programmer receiving coil of the implantable medical device for receiving programmer commands from an external programmer device.

10. The implantable medical device (300) according to any one of claims 1 to 8, **characterised in that** the further receiving device (350) is an antenna of the implantable medical device (300) for telemetry in the MICS frequency band for exchanging data with an external programmer device.

11. The implantable medical device (300) according to any one of claims 1 to 10, **characterised in that** the analysing unit (330) is configured to evaluate a particular response signal in comparison with a reference signal which is stored or was detected in parallel, and to generate a signal indicating the presence of an electrode lead fault if the particular response signal deviates by more than a specified extent.

## Revendications

1. Appareil médical implantable (300) avec un raccord de ligne d'électrode avec au moins un contact électrique pour le raccordement d'une ligne d'électrode (310) et avec une unité d'analyse (330) reliée au raccord de ligne d'électrode qui est conçue pour détecter et exploiter un signal de réponse relié à l'au moins un contact électrique en réaction à un rayonnement électromagnétique (340) **caractérisé en ce que**
l'unité d'analyse (330) est reliée en plus du raccordement à la ligne d'électrode avec un autre dispositif de réception (350) pour une onde électromagnétique irradié et est conçue pour comparer le signal de réponse détecté par le raccord de la ligne d'électrode avec un second signal de réponse reçu par l'autre dispositif de réception pour l'analyse d'erreur.

2. Appareil médical implantable (300) selon la revendication 1, **caractérisé en ce que** l'unité d'analyse (330) est conçue pour détecter un signal de réponse à une irradiation de la bande de fréquences MICS et de l'exploiter.

3. Appareil médical implantable (300) selon la revendication 1, ou 2, **caractérisé en ce que** l'unité d'analyse (330) est conçue pour détecter et exploiter un signal de réponse suite à un rayonnement délivrée par une bobine de programmation d'un appareil de programmation.

4. Appareil médical implantable (300) selon l'une des revendications 1 à 3, **caractérisé en ce que** l'unité d'analyse (330) est reliée, ou est à relier, avec une source de rayonnement électromagnétique, où la source de rayonnement électromagnétique est conçue pour émettre une onde électromagnétique qui est appropriée pour être couplée dans une ligne d'électrode (310) raccordée à un raccord de ligne d'électrode.

5. Appareil médical implantable (300) selon la revendication 4, **caractérisé en ce que** l'unité d'analyse (330) est à relier sans fil avec une source de rayonnement électromagnétique de telle sorte que l'unité d'analyse (330) détecte automatiquement une émission d'une onde électromagnétique par une source de rayonnement respective, où l'unité d'analyse (330) est conçue pour démarrer, en réaction à une détection de l'émission d'une onde électromagnétique par une source de rayonnement respective, une détection et une exploitation d'un signal de réponse correspondant sur l'au moins un contact du raccord de ligne d'électrode.

6. Appareil médical implantable (300) selon la revendication 5, **caractérisé en ce que** l'unité d'analyse (330) est conçue pour reconnaître une parmi plusieurs sources de rayonnement potentielles à l'aide d'un signal émis par la source de rayonnement en question ou à l'aide d'une onde électromagnétique respective émise par une source de rayonnement respective et exploiter de manière spécifique un signal de réponse respectif pour une source de rayonnement respectivement reconnue.

7. Appareil médical implantable (300) selon l'une des revendications 1 à 6, **caractérisé par** une source de rayonnement intégrée dans l'appareil médical implantable.

8. Appareil médical implantable (300) selon l'une des revendications 1 à 7, **caractérisé par** une unité de commande (320) pour la commande de l'appareil médical implantable dans différents modes de fonctionnement, où l'unité de commande (320) est reliée avec l'unité d'analyse (330) et est conçue pour enclencher l'appareil médical implantable (300) dans un mode de fonctionnement dans lequel la ligne d'électrode à tester n'est pas branchée pour la réception des signaux influençant la thérapie, pour la durée de la détection d'un signal de réponse par l'unité d'analyse.

9. Appareil médical implantable (300) selon l'une des revendications 1 à 8, **caractérisé en ce que** l'autre dispositif de réception (350) est une bobine de programmation de l'appareil médical implantable pour la réception de commandes de programmation d'un appareil de programmation externe.

10. Appareil médical implantable (300) selon l'une des revendications 1 à 8, **caractérisé en ce que** l'autre dispositif de réception (350) est une antenne de l'appareil médical implantable (300) pour une télémétrie dans la bande de fréquences MICS pour un échange de données avec un appareil de programmation externe.

11. Appareil médical implantable (300) selon l'une des revendications 1 à 10, **caractérisé en ce que** l'unité d'analyse (330) est conçue pour exploiter un signal de réponse respectif en fonction d'un signal de référence mémorisé ou saisi parallèlement et générer un signal indiquant une erreur de ligne d'électrode lorsque le signal de réponse respectif dévie d'une valeur supérieur à une valeur prédéfinie.
